Europäisches Patentamt

European Patent Office

Office européen des brevets

(11) Publication number: **0 353 987**
A1

# (12) EUROPEAN PATENT APPLICATION

(21) Application number: 89307799.0

(22) Date of filing: 01.08.89

(51) Int. Cl.5: **A 61 K 7/00**
A 61 K 7/06, A 61 K 7/075,
C 08 F 226/00

(30) Priority: 03.08.88 GB 8818444

(43) Date of publication of application:
07.02.90 Bulletin 90/06

(84) Designated Contracting States:
CH DE FR GB IT LI

(71) Applicant: ALLIED COLLOIDS LIMITED
P.O. Box 38 Low Moor
Bradford West Yorkshire, BD12 0JZ (GB)

(72) Inventor: Farrar, David
190 Westfield Lane Idle
Bradford West Yorkshire BD10 8UB (GB)

(74) Representative: Lawrence, Peter Robin Broughton et al
GILL JENNINGS & EVERY 53-64 Chancery Lane
London WC2A 1HN (GB)

(54) Polymers and compositions containing them.

(57) Water-rinsable personal care products such as hair conditioners, conditioning shampoos and skin conditioners include cationic water-soluble polymer formed from a monomer blend that includes cationic monomer and monomer that carries a pendant group $-A_nR$ where n is zero. or a positive integer, A is ethyleneoxy and R is a hydrocarbyl group of 8 to 30 carbon atoms. Polymers of this general type and which contain at least 10% vinyl pyrollidone and/or diallyl dialkyl ammonium halide (for instance DADMAC) are new and are particularly preferred for use in these products.

## Description

### Polymers and Compositions Containing Them

It is well known to thicken aqueous media by the inclusion of what are known as associative thickeners. These are polymers that have pendant hydrophobic groups extending from the polymer backbone. The commercially available associative thickeners, and most publications relating to associative thickeners, are concerned solely with thickeners that are anionic and are made by oil-in-water emulsion polymerisation. Thus these polymers are not water soluble at low or neutral pH but are soluble in alkali. There are therefore used for thickening a wide range of slightly alkaline materials and have been proposed for thickening products as diverse as paints and cosmetics.

Water soluble, anionic or cationic, associative polymers of high molecular weight (single point intrinsic viscosity above 3) are described in EP 172723, primarily for use as flocculants. In EP 216479 there are described polymers that are oil-in-water emulsions and/or are cross linked or are soluble linear polymers having single point intrinsic viscosity above 0.5 and which are characterised by the presence of a particular allyl ether linkage by which the hydrophobic group is connected to the backbone. Some of the polymers are cationic as a result of including within the backbone groups derived from dialkylaminoalkyl -(meth) acrylates and -(meth) acrylamides. That European publication is derived from European application 86306081.0 and cationic polymers containing the allyl ether group are also described in the unprinted priority documents that are in the public file of that European application, for instance in GB 8520218. In that unprinted application, it is stated that the cationic polymers of low or medium molecular weight may be used as cationic surfactant polymers especially as corrosion inhibitors. Elsewhere, that application states

"The polymers may be used in cosmetics and cleaning, including shampoo, compositions. Thus low, medium or high molecular weight anionic, non-ionic or cationic polymers made by reverse phase, oil-in-water or suspension polymerisation may be used as components, for instance thickeners, of face creams and other cosmetics while medium molecular weight anionic and non-ionic polymers made by oil-in-water, reverse phase or solution polymerisation may be used as co-thickening surfactant solutions, for instance in detergents and shampoos."

Accordingly the disclosure in that application is that face creams and other cosmetics can be made using anionic, non-ionic or cationic polymers of the particular allyl ether type, but the only polymers of this type that are proposed for use in detergents and shampoos are anionic and non-ionic polymers. Thus there was no suggestion that the cationic polymers could be used in such compositions and so the proposals in this unprinted application did not alter the general understanding which was that such compositions required the use of anionic associative polymers, for instance as in disclosures of conventional associative polymers, for instance as in EP 13836.

Traditional cosmetics for which associative polymers have been proposed have a high content of oily material and as a consequence the applied film of the cosmetic is relatively hydrophobic and it is not practicable to remove it solely by water rinsing. Surfactant (e.g., soap) is required to assist removal.

An entirely separate class of products are the water-rinsable personal care products that are easily removed solely by water rinsing. These usually contain sufficient surfactant to give a cleaning action. Examples are shampoos, hair conditioners and shaving creams. In order that the composition can provide beneficial properties, even after it has been rinsed off, it is known to include water soluble cationic polymer. This imparts substantivity to anionic surfaces such as skin, hair and nails and so the substantive polymer results in a beneficial effect on such surfaces even after rinsing of the remainder of the composition. Thus the substantive polymer can give a conditioning or softening effect to the surfaces. Examples of such polymers include polymers of diallyl dimethyl ammonium chloride (DADMAC) and copolymers produced from cationic monomers with vinyl pyrollidone.

An important property of the compositions is their physical characteristic before and during use, and in particular their rheology. It is therefore common to include various additives in the composition to try to improve rheology, whilst maintaining substantivity due to the cationic polymer. It is necessary to select additives which are compatible with the polymer and the other essential ingredients of the composition and this can render the formulation of suitable compositions rather complicated and can necessitate the use of a variety of rheology-improving additives in the compositions, and the resultant rheology is still sometimes not entirely satisfactory.

A water-rinsable personal care product according to the invention comprises a cationic, water soluble, polymer formed from a blend of ethylenically unsaturated monomers comprising

(a) 10 to 99% by weight cationic monomer

(b) 1 to 90% by weight of a monomer that carries a pendant group $-A_nR$ where n is zero or a positive integer, A is ethyleneoxy and R is a hydrocarbyl group of 8 to 30 carbon atoms

(c) 0 to 90% by weight non-ionic monomer

(d) 0 to 40% by weight anionic monomer in an amount less than the amount of cationic monomer, i.e., so that the molar proportions of anionic groups and cationic groups are such that the polymer is substantive to anionic surfaces.

Preferably the products of the invention are intended to leave, after water rinsing, a substantive residue on the hair, skin or nails due to the anionic nature of the skin or hair. The polymer is preferably film-forming since this then results in a substantive film being deposited on, for instance, the skin or, especially, the hair.

The presence of monomer (b) in the polymer can improve the perceived benefit of the incorporation of the polymer. Thus the use of the specified polymer can give an improved conditioning effect.

The specified polymer can give significant improvement in the physical characteristics of the composition before or during application and thus can improve the rheology or other physical characteristics. This is of particular benefit when the polymer is also yielding improved properties due to its substantivity. Thus the polymer can be used to stabilise a foam or gel or to increase the viscosity of a liquid or potentially liquid product. The use of the specified polymer, instead of a conventional cationic polymer, can thus give improved conditioning of the hair or skin but, in particular, can result in improved rheology compared to a composition that otherwise has the same ingredients or, particularly, can result in the same or improved rheology without necessitating the addition of a further component to act as a thickening agent. For instance it can be possible to omit some or all of conventional thickeners and to rely instead upon the composition of the invention to promote thickening by an associative effect, especially with surfactant that is present in the composition.

The specified polymers can also have other beneficial effects, for instance improving the cleansing properties of the product as a result of improving surfactant or dispersant properties.

Many of the polymers used in the products of the invention are themselves new materials. The molecular weight of the polymers and the proportions of the monomers will be chosen according to the combination of film forming, performance and rheology properties that is required. For instance the molecular weight is usually at least 10,000 but when film forming or thickening properties are required higher molecular weights, for instance at least 0.5 million and generally 1 million up to, for instance, 5 million or even higher are generally preferred.

A characteristic of the polymers used in the invention is the inclusion of monomer (b) in the monomers from which it is made. Monomers (a), (c) and (d) can be conventional.

Monomer (b) can be an ethylenically unsaturated carboxylic acid ester such as (meth) acrylic ester or itaconic ester but because of the desire for shelf stability under alkaline conditions, it is preferably a monomer that is not easily hydrolysed under these conditions and so is normally a (meth) acrylamide that is substituted on an amide nitrogen by the pendant group or, preferably, is a (meth) allyl ether, for instance as described in EP 216479. The use of (meth) allyl ethers as monomer (b) is particularly advantageous and the combination of such monomers with the preferred monomers (a) (especially DADMAC), leads to novel polymers, and new personal care products, having particularly advantageous storage and other properties.

R is usually alkyl or alkaryl, especially C10 to C24, and any of the values proposed in EP 216479 can be used. n is usually a positive integer of at least 2, and often at least 5, for instance 10 to 100, most preferably 10 to 40.

The amount of the associative monomer (b) is generally at least 5% and preferably at least 10% by weight. Adequate associative and other properties may be achieved if the amount is not more than 30% but the amount can be 50% and in some instances higher amounts are appropriate, e.g., up to 80% by weight.

The cationic monomer can be in free base form but is usually in the form of an acid addition salt or, preferably, a quaternary ammonium salt.

The cationic monomer can be selected from any of the conventional cationic ethylenically unsaturated monomers such as dialkylaminoalkyl (meth) -acrylates or -acrylamides or diallyl dialkyl quaternary ammonium salts such as DADMAC. Because of the need for the product to have good shelf stability, despite being slightly alkaline in many instances, it is particularly preferred that the monomer is selected from dialkylaminoalkyl methacrylates, dialkylamino -$C_{2-8}$ alkyl (meth) acrylamides and diallyl dialkyl quaternary ammonium salts. Preferred cationic monomers are DADMAC, dimethyl amino ethyl methacrylates, dimethyl amino propyl methacrylamide and vinyl pyridine and their acid addition and quaternary salts.

The amount of cationic monomer (a) is generally at least 30% by weight. Frequently it is below 50% but in some instances higher amounts are preferred, e.g., up to 80% by weight.

Monomer (c) can be any non-interfering non-ionic monomer. It can be water insoluble, for instance alkyl (meth) acrylate such as ethyl acrylate, or vinyl acetate, if the synthetic method of preparation permits this. Often however it is water soluble, for instance acrylamide or vinyl pyrollidone.

It is particularly preferred that vinyl pyrollidone should be included in the monomers from which the polymer is made, generally in an amount of from 10 to 90% (often 10 to 50%) by weight of the monomers, since such polymers give particularly advantageous film forming properties and so are especially useful as, for instance, hair conditioners.

The non-ionic monomer (c) can be totally absent but it is often convenient to include such monomer, for instance to improve the film-forming properties or to modify the properties in some other way. Suitable amounts are usually in the range 10 to 50% but higher amounts can be used, e.g., up to 80% by weight.

Suitable monomers for use as monomer (d) are sulphonic and, especially, carboxylic ethylenically unsaturated monomers, especially (meth) acrylic acid.

The anionic monomer (d) is usually absent but small amounts can be present, for instance to improve the compatability of the polymer with anionic surfactant or other anionic components in the product. The molar amount of anionic groups must be sufficiently small, relative to the molar amount of cationic groups, that the polymer behaves as a primarily cationic polymer, for instance the molar amount must not be more than half, and generally not more than a quarter, the amount of cationic groups. Often the amount is not more than 10% by weight of the monomers.

Particularly preferred novel polymers of the invention are polymers formed from monomers (a) (b) and optionally (c) and/or (d), all as described above, wherein the monomers comprise at least one monomer selected from diallyl dialkyl quaternary ammonium salts (especially DADMAC) and vinyl pyrollidone. Thus one type of new polymer is formed from monomers of the general type described above but including at least 10% DADMAC or other diallyl dialkyl quaternary ammonium salt. Thus such polymers can be, for instance, copolymers of the associative monomer (b) with DADMAC alone or with DADMAC and acrylamide or with DADMAC and vinyl pyrollidone. Another type of polymer within this class is a copolymer of the associative monomer (b) with vinyl pyrollidone and a cationic monomer that can be, for instance, dialkyl amino alkyl (meth) acrylate or its quaternary or acid salts.

The polymers are generally linear, most preferably film-forming, polymers and are preferably sufficiently soluble in water that they are present in solution in aqueous water-rinsable compositions of the invention, and in the resultant rinse waters obtained using these compositions. However if desired small amounts of cross linking agent can be incorporated to change the rheology obtainable from the polymers and/or to reduce the solubility of the polymers, for instance as in EP 216479.

The monomer blend is usually water soluble such that the polymers can, if desired, be made by polymerisation while in aqueous solution but water insoluble monomer can be incorporated in the blend, or the blend itself can be water insoluble, in which event the polymer can best be made by oil-in-water emulsion polymerisation, e.g., as described in EP 216479. When the blend is water soluble, the polymer can be made by conventional methods such as reverse phase emulsion or dispersion polymerisation aqueous solution polymerisation, aqueous gel polymerisation or precipitation polymerisation, the particular method being selected according to the molecular weight desired and the monomer types that are being polymerised.

Molecular weight can be regulated in known manner, for instance by the inclusion of a chain transfer agent when low molecular weights are required.

The products of the invention are preferably those in which the cationic polymer can be substantive to anionic surfaces, especially skin or hair, in which event the polymer is preferably film-forming since this then results in a substantive film being deposited on the skin or, especially, hair. The presence of monomer (b), and especially the presence of monomer (b) of the allyl ether type, results in the substantive film or other substantive effect being particularly beneficial.

The personal care products of the invention can be any of the conventional water-rinsable personal care products that are commercially available. One valuable class of such products are for use on the hair and so the product can be a shampoo.

Preferably the product is one that is intended to give long lasting effects, for instance on the hair and so preferably the product has a conditioning action, although it may also have a shampooing action. Preferred products according to the invention therefore include hair shampoos and, especially, conditioners and shampoo conditioners. Such products for the treatment of hair can take the form of viscous liquids or lotions, mousses, creams, foams or gels.

Other personal care products of the invention include shaving products such as foams, creams and gels. Other products can be hydrophilic, water-rinsable, skin cleaners or, especially, conditioners such as viscous liquids foams or gels.

The products of the invention can be in solid form, but generally the products are liquid compositions or potentially liquid compositions such as foams, gels or mousses.

The defined cationic polymer can be the only cationic polymer in the personal care product or it may be used in combination with other conventional cationic polymers for water-rinsable personal care products.

The amount of the defined cationic polymer will be chosen having regard to the properties that it is required to impart but will generally be at least 0.05%, and generally at least 0.1% based on the dry weight of the product. Often it is unnecessary for the amount to be more than 5% but higher amounts, for instance up to 20% or more, e.g., 50%, may be appropriate in some instances.

The products generally have a substantial content of surfactant, e.g., at least 5% and often above 10% and frequently above 20% by weight solids, and indeed some such products may consist substantially entirely of the polymer, surfactant and minor additives such as perfumes. The surfactant can be cationic but is usually non-ionic or, preferably, amphoteric or anionic or mixtures thereof. Any of the surfactants conventionally present in similar personal care products can be used.

When the product does contain anionic components the molar proportions of anionic groups in the surfactant and cationic groups in the polymer (or the excess of cationic groups over anionic groups in the polymer) must be such that coprecipitation of the polymer and/or surfactant does not occur. Coprecipitation is most likely to occur when the molar proportions are similar to one another and so preferably there is a large excess of one over the other. Generally there is a large excess of anionic groups in the composition over cationic groups in the polymer.

The products can be on the acid side of neutral but generally they are slightly alkaline, for instance having a pH above 5 and most usually above 6. The pH is often in the range 6 to 8 although it can sometimes be higher, for instance up to 9 or even higher in some instances.

Fragrances and other conventional additives may be included.

The following are some examples of the invention.

Example 1

183.7g acrylamide solution (49.0%) 141.3g DADMAC solution (63.7%), 20.0g allyl ether of 20 mole ethoxylate of stearyl alcohol and 0.67g sodium EDTA were added to 1655g water contained within a reaction vessel and buffered to pH 6.0 - 6.5 with 1M HCl. This mix was degassed with nitrogen for 1 hour. The bubbler was removed from the liquid but retained within the flask to provide a nitrogen blanket.

The flask was then heated to 55°C and initiated using 250ppm ammonium persulphate based on total aqueous phase. The reation vessel was held at 55°C for 2 hours and then heated to 80°C for 1 hour.

After this time 10g of sodium metabisulphite dissolved in a minimum quantity of water was added and the flask was held at 80°C for a further 60 minutes.

The product was diluted to around 8-9% total solids by adding 352.9g water.

The product dry weight was measured and found to be 8.85%. A Brookfield Viscosity measurement (Spindle 5) gave a value of 208,000 cP.

A control polymer prepared in an identical manner but without the allyl ether monomer gave a Brookfield Viscosity of 6,200 cP at 8.65% solids.

Example 2

A shampoo conditioner is prepared by dissolving this polymer in a minimum amount of water and blending the solution with anionic surfactant of the type conventionally used in hair conditioner shampoos, the amount of polymer being about 0.5% based on the dry weight of the resultant product. Conventional fragrances and other minor additives are included and the product is a viscous liquid. When it is applied to the hair as a conditioner shampoo the cationic polymer is substantive to the hair and so imparts a good conditioning effect. The product has excellent storage stability.

Example 3

A hair conditioner in the form of a clean cream rinse can be made, as a comparison, from 94.6% water, 4% oleyl dimethyl benzyl ammonium chloride, 1% of a copolymer of vinyl pyrollidone and quaternised dimethyl amino ethyl methacrylate and 0.4% of hydroxy ethyl cellulose as a thickener. In the invention, the vinyl pyrollidone copolymer and the hydroxy ethyl cellulose can be omitted and can be replaced by about 1.4% of the polymer of Example 1. The viscosity of the product is at least as high as the viscosity of the product containing the hydroxy ethyl cellulose and the rheology of the product, and the conditioning effect, are generally better.

Example 4

A comparative hair-setting gel can be formed from 1% oleyl alcohol 10 mole ethoxylate, 0.1% of methyl Paraben/propyl Paraben in a 4:1 ratio as preservative, 1% triethanolamine, 0.1% ethylene diamine tetracetic acid sodium salt, 0.1% ultraviolet absorber, minor amounts of colour and perfume, 0.75% cross linked polyacrylic acid as thickener and 7.5% of a polymer of vinyl pyrollidone with quaternised dimethylaminoethyl methacrylate, together with water to make 100%. In the invention, formulation of the composition is simplified and its performance is improved by eliminating the cross linked polyacrylic acid and the vinyl pyrollidone copolymer and replacing them by 7.5% of a polymer of Example 1.

Example 5

A comparative conditioning shampoo is formed from sodium N-coco-N-methyl taurine, 8% lauryl betaine, 2% propylene glycol, 0.1% disodium EDTA, minor amounts of colourant, perfume and preservative, together with 1.2% of a quaternary DADMAC polymer (as indicated in Example 1 as the control product) and, as thickeners, 4% lauric acid diethanolamide and 1.5% ethylene glycol fatty acid esters. In the invention the cationic polymer can be replaced by 2% of the polymer of Example 1 and the thickener blend can be eliminated or significantly reduced without deleteriously affecting performance,

## Claims

1. A water-rinsable personal care product comprising a cationic, water-soluble, polymer formed from a blend of ethylenically unsaturated monomers comprising 10 to 99% by weight cationic monomer, 0 to 90% by weight non-ionic monomer, 0 to 40% by weight anionic monomer in an amount less than the amount of cationic monomer,
characterised in that the blend of monomers includes 1 to 90% by weight of a monomer that carries a pendant group $-A_nR$ where n is zero or a positive integer, A is ethyleneoxy and R is a hydrocarbyl group of 8 to 30 carbon atoms.

2. A product according to claim 1 in which the amount of the cationic monomer is at least 30%, the amount of the non-ionic monomer is at least 10%, the amount of the anionic monomer is 0 to 10%, and the amount of the monomer carrying the pendant group $-A_nR$ is 10 to 50%.

3. A product according to claim 1 or claim 2 in which the monomer that carries the pendant group $-A_nR$ is a (meth) allyl ether.

4. A product according to any preceding claim in which the cationic monomer is selected from dialkylaminoalkyl methacrylates, dialkyamino -C2-8 alkyl (meth) acrylamides and diallyl dialkyl quaternary ammonium salts.

5. A product according to any preceding claim in which the non-ionic monomer is present and is selected from acrylamide and vinyl pyrollidone.

6. A product according to any preceding claim in which the monomers include at least 10% by weight of monomers selected from vinyl pyrollidone and diallyl dialkyl quaternary ammonium salts.

7. A product according to any preceding claim in which the monomers include at least 10% by weight diallyl dimethyl ammonium chloride.

8. A product according to any preceding claim selected from shaving foams, shaving creams, shaving gels, skin conditioners, hair shampoos, hair conditioners and hair shampoo conditioners.

9. A product according to any preceding claim selected from hair conditioners and hair shampoo conditioners.

10. A product according to any preceding claim in which the amount of the said polymer is at least 0.05% dry weight, and the product also includes surfactant in an amount of at least 5% dry weight.

11. A product according to any preceding claim in which the said polymer is film-forming.

12. A cationic, water soluble, polymer formed from a blend of ethylenically unsaturated monomers comprising 10 to 99% by weight cationic monomer, 0 to 90% by weight non-ionic monomer and 0 to 40% by weight anionic monomer in an amount less than the amount of cationic monomer, characterised in that the monomers include 1 to 90% by weight of a monomer that carries a pendant group $-A_nR$ where n is zero or a positive integer, A is ethyleneoxy and R is a hydrocarbyl group of 8 to 30 carbon atoms, and at least 10% by weight of a monomer selected from vinyl pyrollidone and diallyl dialkyl quaternary ammonium salts.

13. A polymer according to claim 12 in which the monomers include at least 10% by weight diallyl dimethyl ammonium chloride.

14. A polymer according to claim 12 or claim 13 and which is film-forming.

15. A polymer according to any of claims 12 to 14 in which the monomer that carries the pendant group $-A_nR$ is a (meth) allyl ether.

## EUROPEAN SEARCH REPORT

Application number

| | DOCUMENTS CONSIDERED TO BE RELEVANT | | | EP 89307799.0 |
|---|---|---|---|---|

| Category | Citation of document with indication, where appropriate, of relevant passages | Relevant to claim | CLASSIFICATION OF THE APPLICATION (Int. Cl.⁴) 5 |
|---|---|---|---|
| A | EP - A1 - 0 208 951 (WELLA AKTIENGESELLSCHAFT) * Claims 1,2; abstract * -- | 1,4,5, 8,9 | A 61 K 7/00 A 61 K 7/06 A 61 K 7/075 C 08 F 226/00 |
| A | EP - A2 - 0 074 819 (AMWAY CORP.) * Page 4, lines 16-22 * -- | 1,4,7, 8,9 | |
| A | EP - A1 - 0 269 243 (CALGON CORP.) * Claims; abstract * -- | 1,4,8, 9 | |
| A | EP - A1 - 0 266 111 (CALGON CORP.) * Claims 1-3; abstract * -- | 1,4,5, 8 | |
| Y | EP - A2 - 0 172 724 (ALLIED COLLOIDS LIMITED) * Claims 1-8 * -- | 12,15 | |
| Y | EP - A2 - 0 126 528 (ALLIED COLLOIDS LIMITED) * Page 7, line 29 - page 8, line 27 * -- | 12 | TECHNICAL FIELDS SEARCHED (Int. Cl.⁴) 5 |
| A | US - A - 4 617 362 (L. W. BECKER et al.) -- | 12,13 | A 61 K 7/00 C 08 F 2/00 C 08 F 20/00 C 08 F 16/00 C 08 F 216/00 C 08 F 226/00 |
| A | EP - A2 - 0 165 770 (ALLIED COLLOIDS LIMITED). -- | 12 | C 08 F 26/00 C 08 F 220/00 |
| A | EP - A1 - 0 188 721 (ALLIED CORP.) -- | 12,13 | |
| A | US - A - 4 579 926 (J. J. MAURER et al.) | 12 | |

The present search report has been drawn up for all claims

| Place of search | Date of completion of the search | Examiner |
|---|---|---|
| VIENNA | 09-11-1989 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | -- | | |
| A | EP - A1 - 0 116 779 (EXXON RESEARCH) | 12 | |
| | -- | | |
| D,A | EP - A2 - 0 172 723 (ALLIED COLLOIDS LIMITED) | 12 | |
| | -- | | |
| D,A | EP - A1 - 0 216 479 (ALLIED COLLOIDS LIMITED) | 12 | |
| | ---- | | |

TECHNICAL FIELDS
SEARCHED (Int. Cl.4)

The present search report has been drawn up for all claims

| Place of search | Date of completion of the search | Examiner |
|---|---|---|
| VIENNA | 09-11-1989 | IRMLER |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO Form 1503 03 82